# EUROPEAN PATENT APPLICATION

(11) **EP 1 202 056 A1**
(43) Date of publication of application: **02.05.2002**
(21) Application number: 00203776.0
(22) Date of filing: 30.10.2000
(51) Int. Cl.: G01N 33/50, G01N 33/566

(54) **Continuous-flow homogeneous bio-chemical assays based on mass spectrometric detection of known ligands**

(71) Applicant: Vrije Universiteit Amsterdam, 1081 HV Amsterdam (NL)
(72) Inventor: Irth, Hubertus, 1081 HV Amsterdam (NL)
(74) Representative: Prins, Adrianus Willem

(57) **Abstract**

The present invention relates to the measurement of interactions, and especially biospecific interactions in an assay using mass spectrometry. More in particular, the present invention makes it possible to measure specific interactions in a homogeneous biochemical assay. The on-line detection method of the present invention comprises contacting an effluent of a fractionation step with a controlled amount of affinity molecules, which affinity molecules bind with analytes suspected to be present in the effluent. This step is followed by the addition of a controlled amount of known ligands, which ligands bind to the affinity molecules, by which ligand/affinity molecule-complexes are formed. In accordance with the invention, the free ligands are detected in the presence of the ligand/affinity molecule-complexes, using a mass spectrometer.

## Description

The present invention relates to the measurement of interactions, and especially biospecific interactions in an assay using mass spectrometry. More in particular, the present invention makes it possible to measure specific interactions in a homogeneous biochemical assay. In a particular embodiment this detection technique is used as monitoring method for biochemical assays using a known ligand. Furthermore, this invention relates to the use of newly detected compounds as ligand for affinity molecules.

Biochemical assays are highly sensitive detection techniques for the measurement of biospecific interactions. The affinity of ligands to be tested for an affinity molecule, *e.g.* an affinity protein such as an antibody, receptor or enzyme is typically tested using a labeled ligand - *i.e.* a compound with known affinity for the affinity molecule such as a protein containing a detectable moiety such as a radiolabel, fluorophor or an enzyme - as reporter molecule.

Biochemical assays are carried out in batch - for example in a microtiterplate - or in continuous-flow. Typically, the affinity molecule is incubated with the sample and a suitable label, and after a certain incubation time, the amount of free or bound label is determined. If the detection properties of free and bound label are identical, both fractions have to be separated before detection. Several homogenous assay formats are known which exploit detection differences between free an bound label, *e.g.* a different fluorescence quantum yield. In such a case, one fraction can be detected in presence of the other avoiding time-consuming and laborious separation steps.

A disadvantage of biochemical assays based on the use of particular labels, is the necessity to find a suitable label for the affinity molecule, such as a protein, to be screened. The synthesis of labels that have sensitive detection properties on the one hand and retain their preferably high binding affinity on the other hand is a difficult task, particularly for receptors where the attachment of a fluorophor or an enzyme to a known ligand often significantly decreases the binding affinity of the label synthesized.

The synthesis of new labels can be avoided if detectors are used that do not require specific chemical or biochemical reporting molecules such as fluorophors or enzymes. A mass spectrometer (MS) is an example of a detector that can measure a compound at the required sensitivity in its native state, *viz.* the compound can be detected without requiring chemical modification. The use of mass spectrometry as a detection technique in biochemical assays therefore overcomes the need to synthesize new labels before the assay can be developed and used.

Seiffert *et al.* (J. Anal. Chem., **363** (1999) 767-770) propose an MS based screening method wherein active ligands are isolated from the sample using an immobilized receptor column. Subsequently, the bound fraction is desorbed and measured by liquid chromatography mass spectrometry. This method represents the direct detection of the active molecule using mass spectrometry. This is particularly disadvantageous for applications wherein novel, unknown ligands have to be detected, since the MS has to be operated in the rather unsensitivite total ion current (TIC) mode. Moreover, the immobilization of receptors may lead to a partial or complete deactivation of the protein due to sterical hindrance. Also, the desorption conditions for the release of the bound active ligands are often leading to an irreversible denaturation of the receptor requiring a frequent exchange of the immobilised receptor column.

Nedved *et al.* (Analytical Chemistry, **68** (1996) 4226-4236) describe a similar technique wherein combinatorial libraries of small molecules are screened using an immobilized immunoaffinity column coupled to reversed-phase liquid chromatography and ion spray mass spectrometry. Again, active compounds are detected directly in the full scan mode. The use of an immobilized antibody column is identical to the above-described method of Seiffert et al. with all disadvantages of this assay format.

Hsieh *et al.* (Molecular Diversity, **2** (1996), 189-196) describe a multidimensional chromatographic screening method using mass spectrometry for detection. After incubation of the affinity protein with the compound(s) to be screened, the bound fraction is separated from the unbound fraction using size-exclusion chromatography. Subsequently, the protein-bound fraction is directed towards a reversed-phase liquid chromatography column coupled to mass spectrometry and the small molecules bound to the affinity protein are determined. In contrast to Seiffert *et al.* and Nedved *et al.*, Hsieh *et al.* describe a biochemical assay wherein the interaction of ligands and receptors takes place in solution, that is the receptor is not immobilized. However, after the primary incubation step, two chromatographic methods have to be applied before the active compound can be detected directly. Moreover, since the structure of the active compound is not known, the mass spectrometer has to be run in the nonselective TIC mode.

The not prepublished EP-A-1 048 951 (Irth and Van der Greef) describes the use of mass spectrometry as a detection technique for measuring biospecific interactions. Rather than using a label, a known ligand with good MS detection properties is used as a reporter molecule to detect the presence of active compounds in a sample. The method described comprises a sequential biochemical reaction, wherein the sample is incubated with an affinity protein, and subsequently a known ligand is added to titrate the remaining free binding sites. In a final step the free fraction of the known ligand is separated from the protein-bound fraction using a hollow-fibre separation module or a restricted access column. Only the free fraction is directed towards the mass spectrometer whereas the protein fraction is discarded. The method of EP-A-1 048 951, hence, is a heterogeneous assay format requiring the separation of the detectable molecule from the protein bound fraction.

The present invention seeks to provide a method that is easier to carry out than heterogeneous assays, which require the separation of free and protein-bound reporter molecules. More particularly, the present invention is aimed at a homogeneous biochemical assay using mass spectrometry as readout method. The assay does not require the separation of free and bound label as described by Irth and Van der Greef. The differences in the method of Irth and Van der Greef and the method of the present invention are shown in Scheme **1**.

In accordance with the present invention, it was found that an on-line detection method wherein an effluent of a fractionation step is contacted with a controlled amount of affinity molecules; followed by a step wherein the affinity molecules are allowed to bind with analytes suspected to be present in the effluent; followed by addition of a controlled amount of known ligands; followed by a step wherein ligands are allowed to bind to the affinity molecules by which ligand/affinity molecule-complexes are formed; and followed by detection of free ligands in the presence of the ligand/affinity molecule-complexes using a mass spectrometer can overcome the aforementioned problems.

Surprisingly, the MS can be used to detect free ligands in the presence of the ligand/affinity protein-complexes, thus obviating the need for cumbersome separation steps. The present invention additionally overcomes a problem associated with separation steps, which problem lies in the nonspecific binding of proteins to surfaces of separation devices such hollow-fiber modules or restricted access phases, which are required in prior art methods, such as the method of Irth and Van der Greef. Moreover, no regeneration of separation devices is required, which allows a continuous operation of assay systems. Furthermore, the time of assay development for new screening assays is significantly reduced since there is no need to study and optimize separation conditions.

The current invention is based on the on-line coupling of an fractionation step such as liquid chromatography to a biochemical reaction module. To the effluent of the fractionation step, a controlled amount of an affinity molecule, e.g. a protein such as a receptor, antibody or enzyme is added. After a short reaction time (maximum about 3 minutes) a controlled amount of a known ligand is added to the reaction mixture and allowed to interact for a short time, *viz.* 2-3 minutes. The entire reaction mixture is introduced into a mass spectrometer without prior separation of free and bound known ligand. The change of amount of known ligand as detected by the MS indicates the presence of an affinity-molecule binding compound in the effluent. In contrast to the heterogeneous assays described by Irth and Van der Greef, the amount of free known ligands is detected in the presence of the bound ligand-affinity protein complex. It is very surprising that free ligand is detected by MS independent from the amount of ligand bound in complexes with affinity molecules.

The method of the present invention makes it possible to detect the presence of an active compound in a mixture by monitoring the changes of the concentration of a known ligand at its specific mass to charge ratio. Moreover, the molecular mass of the active compound can be measured simultaneously by monitoring the total ion current chromatogram at the time where the peak maximum for the known ligand occurs (see scheme 2).

A comparison of the MS data obtained when analytes are injected with the signal obtained when only the controlled amounts of affinity molecule and detectable ligand are introduced in the continuous-flow system, provides information in respect of the percentage of the binding sites occupied by analytes present in the effluent of the fractionation step. The person skilled in the art possesses the knowledge to process and evaluate the data obtained from the detection method. The percentage binding sites occupied by an analyte present in the effluent can be used to find new compounds which show an interaction with a known affinity molecule. This information provides the possibility to implement the on-line separation/affinity molecule detection process of the present invention in, *e.g.*, drug discovery.

A system of high-throughput screening to be used in drug discovery, for instance, consists of the following steps. Complex samples generated for instance by an upstream combinatorial chemistry system are prefractionated in fractions containing compounds of similar polarity using, *e.g.*, a solid-phase extraction technique or electrophoretic sample handling principles. Each fraction may additionally be separated using, *e.g.*, either analytical or preparative-scale liquid chromatographic separation columns. The compounds eluting from said LC column are on-line detected using a suitable affinity molecule detection technique. Where preparative-scale separation columns are applied, a post-column flow-split will be made. One of the two flow streams is subjected to detection using the affinity molecule detection technique; the other stream is directed to a fraction collector. Dependent on the signal-obtained from the affinity molecule detector, fractions containing compounds causing a positive response will be collected while fractions causing a negative response will be discarded. This complete screening method can be automated using known valve-switching processes.

A suitable fractionation method to be used in the methods of the present invention comprises a liquid chromatography separation or a capillary electrophoresis step. Other separation or fractionation techniques which are known to the person skilled in the art and which allow a relatively continuous output stream can, however, be used as well.

In a preferred embodiment, the liquid chromatography separation step is a reversed phase HPLC step.

It will be understood that according to the present invention as the effluent of a fractionation step is also to be understood the effluent of a flow injection, in which a mixture of different compounds is injected. The required selectivity can be obtained by operating the MS such, that selectively tracing of the MS is obtained.

Since the flow injection technique provides a flexible and fast screening method, it is a preferred embodiment of the present invention.

All modes of MS-operation are possible in Flow Injection mode, however, due to the higher background, especially the very selective modes which comprise detecting ions of a selected single m/z trace or of selected multiple m/z traces are suitable.

As already mentioned, the fractionation step can be a liquid chromatography separation, a capillary electrophoresis step or a combinatorial chemistry system. Preferred liquid chromatography fractionation steps comprise HPLC, reversed phase HPLC, capillary electrophoresis (CE), capillary electrochromatography (CEC), isoelectric focusing (IEF) or micellar electrokinetic chromatography (MEKC), all of which techniques are known to the person skilled in the art.

All possible variations in MS techniques known in the art can be profited from according to the present invention. preferably, the MS is of the type chosen from the group consisting of electrospray ionization type, atmospheric pressure ionization type, quadrupole type, magnetic sector type, time-off-flight type, MS/MS, MSⁿ, FTMS type, ion trap type and combinations thereof.

For example, in scanning mode to trace compounds, low resolution MS with all possible instrumental designs of MS can be used, in particular quadrupole, magnetic sector, time-off-flight, FTMS and ion-trap. This generates typically molecular weight data with nominal mass accuracy.

When high resolution MS is applied, using all possible high resolution instrumental designs, in particular magnetic sector, time-off-flight, FTMS and ion trap, molecular weight data with high mass accuracy combined with the elemental composition of the compound can be obtained.

Other known MS techniques comprise tandem MS, such as MS/MS or MSⁿ (for example MS³). Application of these techniques enables the collection of structural information of the ligands which is a preferred embodiment of the present invention. The data in scanning mode can be acquired in data-dependent mode which means that for each peak observed automatically the tandem MS measurement is performed. In addition to this the fragmentation induced in the tandem MS measurement can be coupled by more sophisticated procedures, for example a broad band excitation, which also fragments expected fragments of less importance, such as the [protonated molecule-H₂O]⁺ peak in natural product screening.

The MS can also be operated in single/multiple ion monitoring mode, which can be used for highly selective detection.

Single/multiple ion monitoring mode can be used for highly selective detection. Using low resolution MS in single/multiple ion monitoring mode with all possible low resolution instrumental designs, in particular quadrupole, magnetic sector, time-off-flight, FTMS and ion trap, selective detection based on monitoring of previously determined m/z trace can be obtained. When high resolution MS is applied all possible instrumental designs of MS can be used, especially quadrupole, magnetic sensor, time-off-flight, FTMS and ion-trap. This enables typically very selective detection based on monitoring in high resolution previously determined m/z trace. When tandem MS is applied, all possible instrumental designs, in particular ion trap, quadrupole-time-off-flight (Q-TOF), triple quadrupoles (QQQ), FTMS and combinations of sector instruments with quadrupoles and ion traps, can be used. This enables the very selective detection based on monitoring a resulting peak or peaks in MS/MS and/or MSⁿ measurements.

It is also possible to operate the MS in scanning mode. In this way the lowering of the pre-determined signal is correlated to the increase of other signals, enabling the active compound to be characterized in the same cycle.

With the assays according to the present invention the tracing of (bio-) active compounds in solutions of complex nature, for instance biological fluids or extracts, natural product extracts, solutions or extracts from biotechnological processes, resulting from chemical experiments, such as combinatorial technologies and processes and the like can be performed with a higher efficiency, selectivity and flexibility. Moreover, the present invention provides the possibility to limit the disturbance of background compounds.

The present invention further enables the identification of compounds based on conventional mass spectra, high resolution data or MSⁿ based spectra.

With the method of the present invention it is also possible to perform library searching, based on a variety of mass spectrometric experiments enabling the screening of large series of samples and classifying these in classes based on similar active compounds, without the need for full identification of the compounds.

The assay method of the present invention can be applied in miniaturized formats of assay-based systems, for instance with chip technology based screening systems.

In the methods of the present invention all molecules capable of interaction with or binding to other molecules can be used as affinity molecule. The affinity molecule can for example be selected from the group of cytosolic receptors, *e.g.,* the estrogen, glucocorticoid receptors; solubilized membrane bound receptor, *e.g.,* a β-receptor; affinity proteins, such as antibodies, enzymes, avidin; polynucleotides and polysaccharides.

By a "controlled amount of affinity molecule" that is added to the effluent is to be understood an amount of known concentration and of known flow rate.

The term "known ligand" as used in the present description and claims refers to a ligand which is capable of interacting or reacting with the above-defined affinity molecule, and which can be detected by the MS. An example of a detectable ligand is a previously found analyte capable of being bound by the affinity molecule.

The present invention has the great advantage that the preparation of any label is not required. This is particularly advantageous for those affinity molecules where labeling of a known ligand leads to a dramatic loss of affinity, for example due to steric hindering. Assays using known, natural, ligands without labels as reporter molecules are much faster to develop.

On-line coupling as used in the methods of the present invention requires fast reaction times in order to minimize extra-column band broadening. This means that affinity molecule-ligand interactions having reaction times in the order of minutes rather than hours should be considered. The suitable binding conditions under which the affinity molecules bind to the analyte comprise a contact time, which is in the same order of magnitude. Suitable binding conditions are conditions that provide optimal binding between the affinity molecules and the analyte. It will be understood that the precise conditions, such as temperature, residence time, chemical composition, will depend strongly on the type of assay and the proteins used therein. In contrast to biochemical assays based on fluorescent, radioactive or enzyme labels, where background buffer solutions are mainly composed of involatile phosphate or tris buffers the homogeneous mass spectrometric biochemical assays presented here are performed using volatile buffers such as ammonium formate or ammonium acetate at neutral pH. Moreover, to improve the ionization characteristics of the known ligand, small amounts of methanol (2.5-10%) are added to the background buffer.

In another preferred embodiment, combinations of affinity proteins and known ligands can be used to screen several biomolecular targets at the same time (see scheme 3). For this purpose, a mixture of affinity proteins is prepared and added to the effluent of the fractionation method. Subsequently a mixture of known ligands, containing at least one active known ligand per affinity protein, is added. The reaction mixtures is directed towards the MS. In the MS each active known ligand is monitored by selected ion monitoring (SIM). The presence of an active compound in the fractionation effluent leads to a change of signal of the corresponding known ligand trace in the mass spectrometer. According to this preferred embodiment of the method of the invention, the affinity molecules are thus present in a mixture of two or more different types of affinity molecules and the known ligands are present in a mixture of different ligands, while each of this ligands is known to bind to one of said affinity molecules.

In another preferred embodiment, the on-line biochemical assay is coupled to a multiple-inlet unit for the mass spectrometer (see scheme 4). The multiple-inlet unit allows the connection of one mass spectrometer to multiple (*e.g.* eight) different fractionation lines. In each line an individual known ligand assay is carried out. This methodology is particularly useful for the screening of similar affinity proteins. It allows a multitude of assays to be performed simultaneously without affecting each other (for example by active compounds which react with several affinity proteins). In this way on-line biochemical assays can be performed in parallel using a single mass spectrometer as detector.

### Brief description of the drawings

Scheme 1 is a schematic representation of the on-line assay according to the invention, compared with the method of Irth and Van der Greef (EP-A-1 048 951).

Scheme 2 shows a typical chromatogram obtained with the method of the invention. The upper chromatogram (MS signal vs. retention time) is obtained by screening the effluent in selected ion monitoring mode (SIM), *viz.* the MS only screens for a value of m/z which is typical for the known ligand, L. The chromatogram indicates an increase in free ligand concentration, [L], at a certain retention time, which is indicative for the presence of a compound (analyte) that binds to the affinity molecule. This peak is also measured in total ion current mode (middle diagram of Scheme 2). The full mass spectrum at the retention time corresponding to the peak in [L] is given in the lower diagram of Scheme 2. From this spectrum structural information on the analyte can be obtained.

Scheme 3 shows a preferred embodiment of the invention, wherein combinations of affinity proteins and known ligands are used to screen several biomolecular targets at the same time.

Scheme 4 shows a preferred embodiment which uses a multiple-inlet unit for the mass spectrometer.

Figure 1 shows the results obtained with Example 1; the MS signal in time at m/z 390.

Figure 2 shows the results obtained for biotinylated analytes (Experiment 1).

Figure 3 shows the selected-ion monitoring MS signal at m/z 408 (Experiment 2).

### Example 1

The feasibility of a homogeneous, on-line mass spectrometric biochemical assay was demonstrated using streptavidin as affinity protein, fluorescein-biotin as reporter molecules and biotinylated compounds as analytes. Streptavidin is a well-known affinity protein with a high affinity for biotin and biotinylated compounds. To a carrier solution consisting of 10 mmol/L ammonium formate/methanol (90:10, v/v) pumped at a flow rate of 50 µl/min a solution of 32 nmol/l streptavidin dissolved in 10 mmol/L ammonium formate was added at a flow rate of 50 µl/min. The mixture was pumped through a knitted 300 mm i.d. poly(tetrafluoroethylene) reaction coil with an internal volume of 17 µl. Subsequently, a solution of 96 nmol/l fluorescein-biotin dissolved in 10 mmol/L ammonium formate/methanol (90:10, v/v) was added at a flow rate of 100 µl/min. The mixture was then pumped through a knitted 300 mm i.d. poly(tetrafluoroethylene) reaction coil with an internal volume of 33 µl and subsequently introduced into a mass spectrometer equipped with an electrospray ionization (ESI) source. Mass spectrometry was operated in selected-ion monitoring (SIM) in the positive-ion (PI) mode on [M+H]⁺ and on one or more intense fragment ions. The ESI source conditions for the streptavidin/biotin system: source temperature, 80°C; capillary voltage, 3.2 kV. The sampling cone voltage was 30 and 60 V for biotin and fluorescein-labeled biotin, respectively.

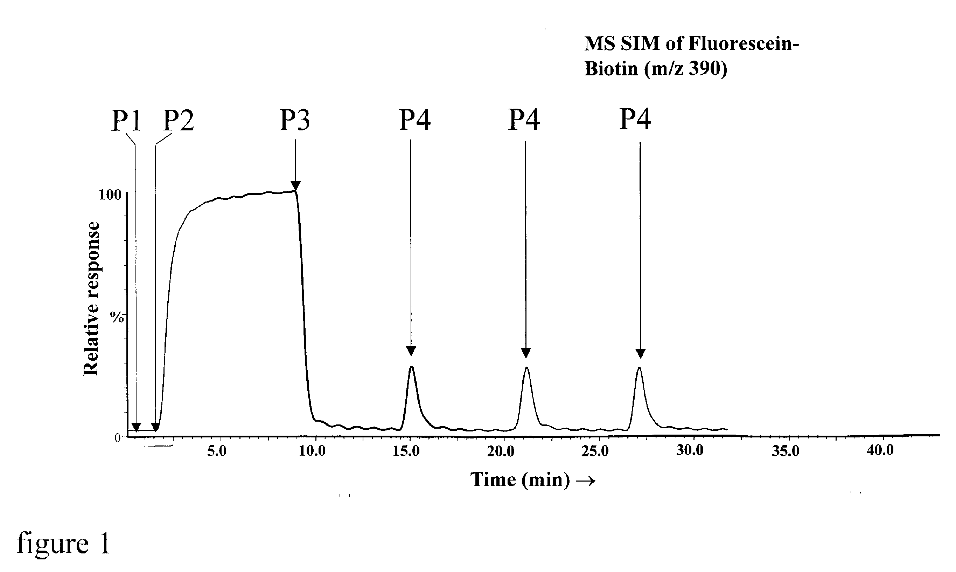

Figure 1 demonstrates an experiment, wherein the entire reaction sequence can be followed. At point 1, only carrier buffer was pumped into the mass spectrometer providing a reference background signal. At point 2, the fluorescein-biotin pump was switched on leading to an increase of the selected-ion monitoring MS signal at m/z 390. At point 3, the streptavidin pump was switched on. The addition of the affinity protein streptavidin lead to a decrease of the concentration of free fluorescein-biotin, resulting in a decrease of selected-ion monitoring MS signal. At point 4, injections of biotin were performed, resulting in a decrease of free streptavidin binding sites and an increase of the free fluorescein-biotin concentration, which can be seen as a peak in selected-ion monitoring MS signal at m/z 390.

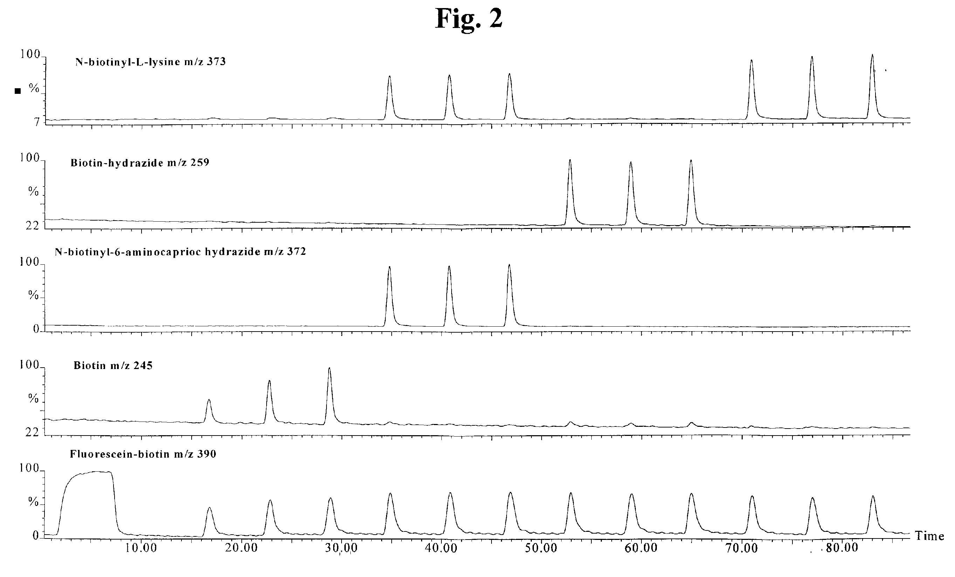

Figure 2 demonstrates the screening method was selective for biotinylated analytes. Injections of equal concentrations of biotin (m/z 245), N-biotinyl-6-aminocaprioc hydrazide (m/z 372), biotin-hydrazide (m/z 259), and N-biotin-L-lysine (m/z 373) lead to almost equal signals at the fluorescein-biotin (m/z 390) trace, indicating that all compounds are capable to prevent the binding of the reporter molecule, fluorescein-biotin, to streptavidin. Furthermore, the molecular mass of the active compounds could be identified for those compounds which are ionized under the selected conditions.

### Example 2

The feasibility of a homogeneous, on-line mass spectrometric biochemical assay for the measurement of weaker interactions than streptavidin/biotin was demonstrated using fab fragments of anti-digoxigenin antibodies (fab anti-digoxigenin) as affinity protein, digoxigenin as reporter molecules and digoxin as analytes. To a carrier solution consisting of 10 mmol/L ammonium formate/methanol (90:10, v/v) pumped at a flow rate of 50 µl/min a solution of 100 nmol/l fab anti-digoxigenin dissolved in 10 mmol/L ammonium formate was added at a flow rate of 50 µl/min. The mixture was pumped through a knitted 300 µm i.d. poly(tetrafluoroethylene) reaction coil with an internal volume of 65 µl. Subsequently, a solution of 200 nmol/1 digoxigenin dissolved in 10 mmol/L ammonium formate/methanol (90:10, v/v) is added at a flow rate of 100 µl/min. The mixture was then pumped through a knitted 300 µm i.d. poly(tetrafluoroethylene) reaction coil with an internal volume of 33 µl and subsequently introduced into a mass spectrometer equipped with an electrospray ionization (ESI) source. Mass spectrometry was operated in selected-ion monitoring (SIM) in the positive-ion (PI) mode on [M+H]⁺ and on one or more intense fragment ions.

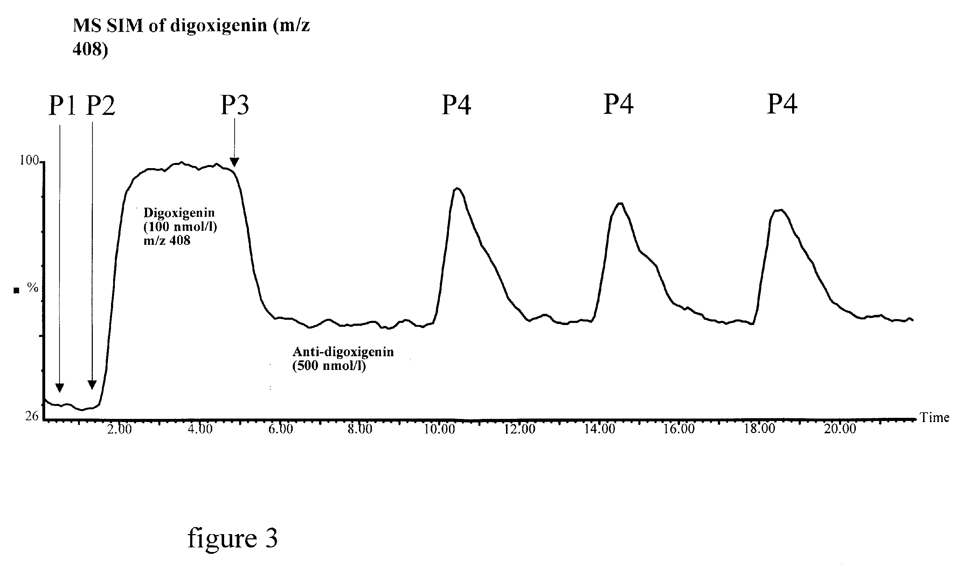

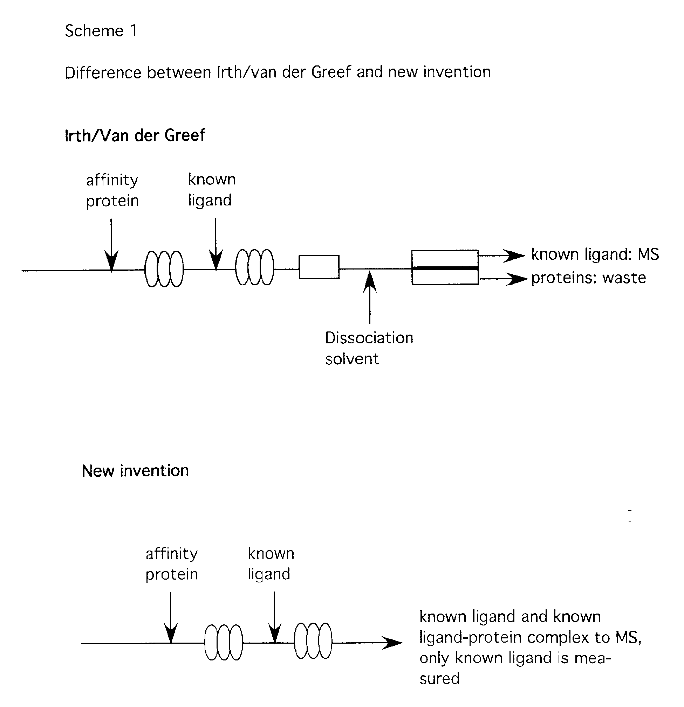

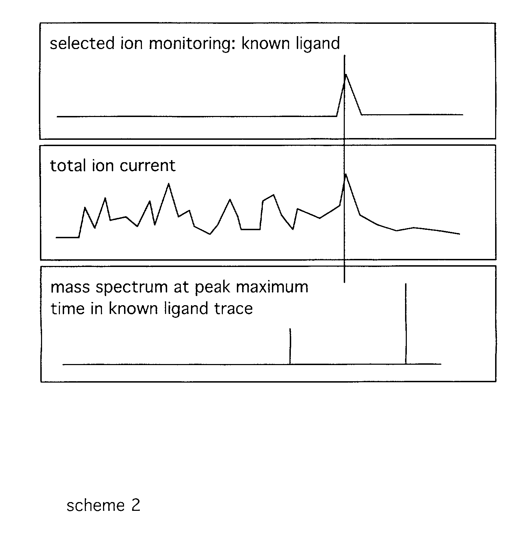

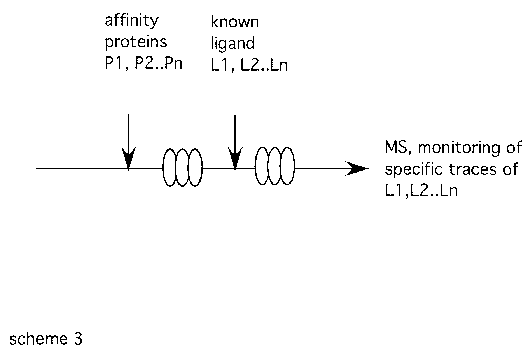

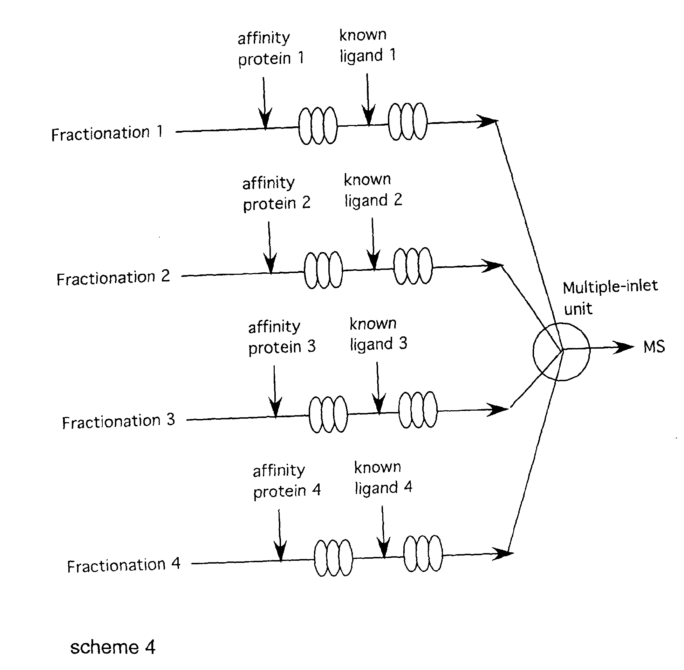

Figure 3 demonstrates an experiment, where the entire reaction sequence can be followed. At point 1, only carrier buffer was pumped into the mass spectrometer providing a reference background signal. At point 2, the digoxigenin pump was switched on leading to an increase of the selected-ion monitoring MS signal at m/z 408 (the ammonium adduct of digoxigenin). At point 3, the fab anti-digoxigenin pump was switched on. The addition of the affinity protein fab anti-digoxigenin lead to a decrease of the concentration of free digoxigenin, resulting in a decrease of selected-ion monitoring MS signal. At point 4, injections of digoxin were performed, resulting in a decrease of free fab anti-digoxigenin binding sites and an increase of the free digoxigenin concentration, which can be seen as a peak in selected-ion monitoring MS signal at m/z 408.

## Claims

1. On-line detection method comprising the steps of: contacting an effluent of a fractionation step with a controlled amount of affinity molecules; allowing the affinity molecules to bind with analytes suspected to be present in the effluent; addition of a controlled amount of known ligands; allowing ligands to bind to the affinity molecules by which ligand/affinity molecule-complexes are formed; and detection of free ligands in the presence of the ligand/affinity molecule-complexes using a mass spectrometer.

2. Method according to claim 1, wherein said fractionation step is a liquid chromatography separation, a capillary electrophoresis step or a combinatorial chemistry system.

3. Method according to claim 2, wherein the liquid chromatography separation step is an HPLC, a reversed phase HPLC, a CE, a CEC, an IEF or an MEKC step.

4. Method according to any one of the preceding claims, using a mass spectrometer selected from the group consisting of electrospray ionization type, atmospheric pressure ionization type, quadrupole type, magnetic sector type, time-off-flight type, MS/MS, MSⁿ, FTMS type, ion trap type and combinations thereof.

5. Method according to any of the previous claims, wherein said affinity molecules are present in a mixture of two or more different types of affinity molecules and said known ligands are present in a mixture of different ligands, each of which ligand is known to bind to one of said affinity molecules.

6. On-line detection method, wherein a mass spectrometer with a multiple-inlet unit is used, to which multiple-inlet unit different fractionation lines are connected, wherein each fractionation line comprises an effluent to which controlled amounts of affinity molecules and known ligands are added as described in each of the previous claims.

7. Compound detected by the method of any one of the preceding claims.

8. Use of the compound of claim 7 as a ligand for affinity molecules.
